Europäisches Patentamt

European Patent Office

Office européen des brevets

(19)

(11) Veröffentlichungsnummer: **0 011 231**
**B1**

(12) # EUROPÄISCHE PATENTSCHRIFT

(45) Veröffentlichungstag der Patentschrift:
02.06.82

(51) Int. Cl.³: **C 07 G 7/00,** A 61 K 35/16, A 61 K 37/04, A 61 K 39/395

(21) Anmeldenummer: 79104365.6

(22) Anmeldetag: 07.11.79

(54) Verfahren zur Herstellung des kälteunlöslichen Globulins und dieses enthaltende Arzneimittel.

(30) Priorität: 09.11.78 DE 2848529

(43) Veröffentlichungstag der Anmeldung:
28.05.80 Patentblatt 80/11

(45) Bekanntmachung des Hinweises auf die Patenterteilung:
02.06.82 Patentblatt 82/22

(84) Benannte Vertragsstaaten:
AT CH DE FR GB IT SE

(73) Patentinhaber: BEHRINGWERKE
AKTIENGESELLSCHAFT, Postfach 1140,
D-3550 Marburg/Lahn (DE)

(72) Erfinder: Schwinn, Horst, Dr., Stümpelstal 27,
D-3550 Marburg/Lahn (DE)
Erfinder: Helmburger, Norbert, Dr., Sonnenhang 10,
D-3550 Marburg/Lahn (DE)
Erfinder: Kumpe, Gerhard, Aspherfeld 14, D-3552 Wetter
(DE)
Erfinder: Herchenhan, Bernd, Im Brand 33,
D-3570 Kirchhain (DE)

(74) Vertreter: Meyer-Dulheuer, Karl-Hermann, Dr. et al,
HOECHST Aktiengesellschaft Zentrale Patentabteilung
Postfach 80 03 20, D-6230 Frankfurt/Main 80 (DE)

(56) Entgegenhaltungen:
DE A 2 715 832
Chemical Abstracts Band 74, Nr. 3, 18. Januar 1971
Columbus, Ohio, USA, M. W. MOSESSON et al.
»Cold-insoluble globulin of human plasma. I.
Purification, primary characterization, and relation of fibrinogen and other cold-insoluble fraction
components« Seite 15, Spalte 1, Abstract Nr. 9658f
Chemical Abstracts Band 83, Nr. 21, 24. November
1975 Columbus, Ohio, USA, D. F. MOSHER
»Cross-linking of cold-insoluble globulin by fibrinstabilizing factor« Seite 346, Spalte 2 bis Seite
347, Spalte 1, Abstract Nr. 176110p

Chemical Abstracts Band 86, Nr. 23, 6. Juni 1977
Columbus, Ohio, USA, A. B. CHEN et al. »An
improved method for purification of the cold-insoluble globulin of human plasma (Cig)« Seite 235,
Spalte 2, Abstract Nr. 167439n

Verfahren zur Herstellung des kälteunlöslichen Globulins und dieses enthaltende Arzneimittel

Die Erfindung betrifft ein Verfahren zur Herstellung des kälteunlöslichen Globulins, vor allem aus Faktor VIII-Konzentraten des Blutplasmas, sowie ein dieses enthaltendes Arzneimittel.

Das kälteunlösliche Globulin (=cold insoluble globulin, CIG), ist als ein im Plasma vorkommendes Protein unter dem Namen »large external transformation-sensitive protein« = LETS, »soluble firoblast antigen« = SF, »cell surface protein« = CSP, »cell adhesion factor« = CAF oder Fibronectin und als Bestandteil der Oberfläche von Fibroblasten, seit längerem in der Literatur beschrieben.

Es ist bereits bekannt, daß dieses Protein aus der Cohn I-Fraktion bzw. dem Kryopräzipitat von ACD (Acid citric dextrose)-Plasma in präparativen Mengen gewonnen werden kann (Mosesson, M. W. und Umfleet, R. A., J. Biol. Chem. 245, 5728 [1970]). Die Autoren gehen dabei von einem Ausgangsmaterial aus, welches neben anderen kälteunlöslichen Spurenproteinen hauptsächlich Fibrinogen (80–85%) und das CIG (max. 10%) enthält.

Das Problem der CIG-Präparation beruht in erster Linie in der Abtrennung vom Fibrinogen, dem es in seinen Fällungs- und Fraktioniereigenschaften sehr ähnlich ist. Dies wurde von Mosesson (s. o.) durch eine Fraktionierung mit Glycin/Äthanol bei niedrigen Temperaturen versucht.

Das auf diese Weise angereicherte Protein enthält aber immer noch ca. 50% Fibrinogen, welches erst durch einen nachgeschalteten lonenaustauscher-Schritt ausreichend entfernt werden kann.

Es ist weiterhin bekannt, daß die kommerziell erhältlichen Faktor VIII-Konzentrate, weitgehend unabhängig von Herkunft und Herstellungsverfahren, Fibrinogen und CIG in relativ großen Konzentrationen und unterschiedlichen Verhältnissen als Begleitproteine enthalten.

Nun wurde überraschend gefunden, daß es möglich ist, CIG aus dieses enthaltenden fibrinogenarmen Faktor VIII-Konzentraten mit wenigen Fraktionierschritten in hoher Reinheit herzustellen. Dabei fällt einerseits CIG, andererseits Faktor VIII an.

Gegenstand der Erfindung ist demnach ein Verfahren zur Herstellung von kälteunlöslichem Globulin (CIG) aus dieses enthaltenden, vorteilhaft fibrinogenarmen Faktor VIII-Konzentraten, dadurch gekennzeichnet, daß man

a)  dieses bei einer Temperatur >18°C mit 1,8–2,6 mol/l einer wasserlöslichen aliphatischen Aminosäure und 8–12% (w/v) Neutralsalz versetzt und

b)  aus dem Überstand mit 12–33% (w/v) Neutralsalz das CIG ausfällt.

Nach dem beschriebenen Verfahren lassen sich auch CIG-freies Faktor VIII-Konzentrat und CIG gleichzeitig aus demselben Ausgangsmaterial herstellen.

Die Auswahl des geeigneten Ausgangsmaterials erfolgt aufgrund der Bestimmung des CIG, z. B. nach Mosesson and Umfleet, J. Biol. Chem. 245, 5728 (1970), des Faktors VIII, z. B. nach Proctor, M. and Rapaport, O., Am. J. Clin. Paht. 36, 212 (1961) und des Fibrinogens, z. B. nach Clauss, A., Acta haemat. 17, 237 (1957).

Das erfindungsgemäße Verfahren geht von CIG-enthaltenden Faktor VIII-Konzentraten aus, vorzugsweise von fibrinogenarmen Faktor VIII-Konzentraten, insbesondere von solchen, die 0–2 mg Fibrinogen/20 Einh. Faktor VIII enthalten.

Geeignete Faktor VIII-Konzentrate sind an Faktor VIII angereicherte Plasmaproteinfraktionen. Sie sind z. B. erhältlich nach Kryopräzipitationsverfahren oder Fällungsverfahren mit Aminosäuren und können gewünschtenfalls lyophilisiert werden.

Beispielsweise kann ein lyophilisiertes Faktor VIII-Konzentrat in einem 0,02–0,12 M Puffer, vorzugsweise Citrat-NaCl-Puffer, 0,08 mol/l, von pH-Wert 6,0–8,0, vorzugsweise 6,9, in einer Konzentration von 0,2–20%, vorzugsweise 1%, bezogen auf Protein, gelöst werden.

Bei einer Temperatur oberhalb 18°C, jedoch begrenzt durch die Denaturierung bei hohen Temperaturen, vorzugsweise bei 20–25°C, wird 1,8–2,6, vorzugsweise 2,2 mol/l einer wasserlöslichen aliphatischen Aminosäure, vorzugsweise Glycin und 8–12%, vorzugsweise 12% w/v Neutralsalz, insbesondere NaCl, in festem Zustand zugesetzt. Die Fällung wird abgetrennt, vorzugsweise abzentrifugiert, sie kann gewünschtenfalls zur Faktor VIII-Herstellung weiterverwendet werden.

Der Überstand wird mit Neutralsalz auf einen Salzgehalt von etwa 12–33% w/v, vorzugsweise 17% w/v gebracht. Geeignete Neutralsalze sind Ammoniumsulfat aber vorzugsweise Alkalihalogenide mit ausreichender Löslichkeit in Wasser.

Der Niederschlag wird, wie oben beschrieben, gewonnen. Zur weiteren Anreicherung kann er in einer dem Ausgangsvolumen gleichen oder einer geringeren Menge Pufferlösung, vorzugsweise einer Lösung des genannten Citratpuffers aufgenommen und der vorher beschriebene Fällungsvorgang wiederholt werden.

Abhängig von der gewünschten Reinheit des Produktes kann dieser Fällungsschritt mehrmals wiederholt werden, er kann aber auch wegfallen. Das Endprodukt soll mindestens 60% CIG, bezogen auf Eiweiß enthalten und frei von mit Thrombin gerinnbarem Protein sein. Die Reinheit des Endproduktes wird mit den gängigen proteinanalytischen Methoden wie Polyacrylamidelektrophorese oder Immunelektrophorese unter Verwendung von Standards bzw. spezifischen Antiseren auf begleitendes Gammaglobulin und Fibrinogen hin überprüft.

CIG ist ein wertvolles Arzneimittel. Es kann insbesondere zur Schocktherapie eingesetzt werden. Experimentell läßt sich dies an drei Tiermodellen zeigen:

a) in der passiv-cutanen Anaphylaxie-Reaktion beim Meerschweinchen;

b) im Endotoxinschock durch Lipopolysaccharid bei der Katze;

c) im Histaminschock beim Meerschweinchen.

Mit einer intravenösen Gabe von 10 mg/kg CIG vor oder nach Herbeiführung des Schockzustandes kann die Schockwirkung vermindert werden bzw. die zur Schockauslösung erforderliche Dosis des Histamins bzw. Endotoxins beträgt das ca. 3fache der Kontrolle.

Das CIG wird als Arzneimittel in an sich bekannter Weise für ein Präparat zur parenteralen, vorzugsweise intravenösen Application zubereitet. Es liegt dazu in einer Konzentration von ca. 10 mg/ml in einer wäßrigen Lösung oder in getrockneter, vorzugsweise gefriergetrockneter Form vor. Zur Stabilisierung der CIG-Aktivität verwendet man zweckmäßig entsprechende Zusätze wie Kohlehydrate, in Wasser gut lösliche Aminosäuren und Puffersalze wie Glucose, Glycin und Na-Citrat. Das Präparat ist auch zur Steigerung der opsonierenden Wirkung von phagozytierenden Zellen geeignet.

Die Erfindung soll an dem nachstehenden Beispiel näher erläutert werden:

### Beispiel

Fibrinogenarmes Faktor-VIII-Konzentrat, erhalten nach K. M. Brinkhous et al., JAMA, 105, 67 (1968) oder M. Wickerhauser, Vox. Sang., 23, 402 (1972), wird in einem Citrat-NaCl-Puffer, pH 6,9, 0,02 bzw. 0,06 mol/l, zu einer 1%igen Lösung, bezogen auf Protein, gelöst. Unter Rühren werden bei +22°C zuerst 2,2 mol/l Glycin und dann 12% w/v NaCl, jeweils in festem Zustand portionsweise zugesetzt. Man rührt 2 Stunden bei dieser Temperatur weiter und zentrifugiert sodann die Fällung bei 3000 g während 30 Minuten ab. Der Niederschlag kann als Faktor VIII-Konzentrat weiterverarbeitet werden.

Der Überstand wird auf +37°C erwärmt. Unter Rühren wird dazu festes NaCl bis zu einer Konzentration von 17% w/v gegeben. Man rührt 30 Minuten bei 37°C weiter und gewinnt die Ausfällung durch Zentrifugieren bei 3000 g in 30 Minuten. Der Überstand wird verworfen.

Der Niederschlag wird in 1/4 Volumen des ursprünglich verwendeten Citrat-NaCl-Puffers aufgenommen, wieder auf 37°C erwärmt und mit 2,2 mol/l Glycin und 17% w/v NaCl versetzt und abermals zentrifugiert.

Der dabei erhaltene Niederschlag wird in 1/10 des ursprünglich verwendeten Volumens des Citrat-NaCl-Puffers aufgenommen und gegen das 30fache Volumen dieses Puffers 3 Stunden dialysiert. Danach wird die Lösung auf 30°C erwärmt und zur Klärung 60 Minuten bei 3000 g zentrifugiert.

Der das CIG enthaltende Überstand wird mit Citrat-NaCl-Puffer auf eine Konzentration von 1%, bezogen auf Protein, verdünnt, mit 5% w/v Glucose versetzt, sterilfiltriert und gefriergetrocknet.

### Patentansprüche der Vertragsstaaten: CH, DE, FR, GB, IT, SE

1. Verfahren zur Herstellung des kälteunlöslichen Globulins (CIG), dadurch gekennzeichnet, daß man ein dieses enthaltendes Faktor VIII-Konzentrat bei einer Temperatur >18°C mit 1,8−2,6 mol/l einer wasserlöslichen aliphatischen Aminosäure und 8−12% (w/v) Neutralsalz versetzt, den Überstand von der entstehenden Fällung abtrennt und in diesem die Konzentration des Neutralsalzes weiter erhöht, wobei das CIG ausfällt.

2. Verfahren nach Anspruch 1, dadurch gekennzeichnet, daß als Aminosäure Glycin verwendet wird.

3. Verfahren nach Anspruch 1 und 2, dadurch gekennzeichnet, daß zur weiteren Anreicherung des CIG die Fällung in einer Pufferlösung aufgenommen und erneut unter den Bedingungen des Anspruchs 1 gefällt wird.

4. Arzneimittel zur parenteralen Verabreichung, gekennzeichnet durch einen Gehalt des nach Anspruch 1 erhaltenen CIG.

### Patentansprüche für den Vertragsstaat: A

1. Verfahren zur Herstellung des kälteunlöslichen Globulins (CIG), dadurch gekennzeichnet, daß man ein dieses enthaltendes Faktor VIII-Konzentrat bei einer Temperatur >18°C mit 1,8−2,6 mol/l einer wasserlöslichen aliphatischen Aminosäure und 8−12% (w/v) Neutralsalz versetzt, den Überstand von der entstandenen Fällung abtrennt und in diesem die Konzentration des Neutralsalzes weiter erhöht, wobei das CIG ausfällt.

2. Verfahren nach Anspruch 1, dadurch gekennzeichnet, daß als Aminosäure Glycin verwendet wird.

3. Verfahren nach Anspruch 1 und 2, dadurch gekennzeichnet, daß zur weiteren Anreicherung des CIG die Fällung in einer Pufferlösung aufgenommen und erneut unter den Bedingungen des Anspruchs 1 gefällt wird.

### Claims for the Contracting states: CH, DE, FR, GB, IT, SE

1. A process for the preparation of the cold insoluble globulin (CIG), which comprises adding to a CIG-containing factor VIII concentrate at a temperature above 18°C of from 1.8 to 2.6 mols/l of a water-soluble aliphatic amino acid and of from 8 to 12 w/v % of neutral salt, separating the supernatant from the resulting precipitate and increasing the concentration of neutral salt in the supernatant to make the CIG precipitate.

2. The process as claimed in claim 1, which comprises using as amino acid glycin.

3. The process als claimed in any one of claims 1 and 2, which comprises dissolving the precipitate in a buffer solution for further enrichment of the CIG and carrying out precipitation under the conditions of claim 1.

4. Medicament for the parenteral administration, characterized by a content of CIG prepared according to claim 1.

**Claims for the Contracting state: AT**

1. A process for the preparation of the cold insoluble globulin (CIG), which comprises adding to a CIG-containing factor VIII concentrate at a temperature above 18°C of from 1.8 to 2.6 mols/l of a water-soluble aliphatic amino acid and of from 8 to 12 w/v % of neutral salt, separating the supernatant from the resulting precipitate and increasing the concentration of neutral salt in the supernatant to make the CIG precipitate.

2. The process as claimed in claim 1, which comprises using as amino acid glycin.

3. The process as claimed in any one of claims 1 and 2, which comprises dissolving the precipitate in a buffer solution for further enrichment of the CIG and carrying out precipitation under the conditions of claim 1.

**Revendications pour les États contractants: CH, DE, FR, GB, IT, SE**

1. Procédé de préparation de globuline insoluble au froid (CIG), caractérisé en ce qu'on traite un concentré de Facteur VIII contenant celle-ci, à une température supérieure à 18°C, avec de 1,8 à 2,6 moles/l d'un acide aminé aliphatique soluble dans l'eau et de 8 à 12% en poids/volume d'un sel neutre, on sépare le produit surnagenat du précipité formé et, dans le produit surnageant, on précipite la CIG en augmentant encore la concentration en sel neutre.

2. Procédé selon la revendication 1, caractérisé en ce que, comme acide aminé, on utilise la glycine.

3. Procédé selon la revendication 1 ou 2, caractérisé en ce que, pour un autre enrichissement en CIG, on reprend le précipité dans une solution tampon et on précipite de nouveau dans les conditions de la revendication 1.

4. Médicament pour l'administration parentérale, caractérisé en ce qu'il contient de la CIG obtenue par le procédé selon la revendication 1.

**Revendications pour l'État contractant: AT**

1. Procédé de préparation de globuline insoluble au froid (CIG), caractérisé en ce qu'on traite un concentré de Facteur VIII contenant celle-ci à une température supérieure à 18°C avec de 1,8 à 2,6 moles/l d'un acide aminé aliphatique bien soluble dans l'eau et de 8 à 12% en poids/volume d'un sel neutre, on sépare le produit surnageant du précipité formé et, dans le produit surnageant, on précipite la CIG en augmentant encore la concentration en sel neutre.

2. Procédé selon la revendication 1, caractérisé en ce que, comme acide aminé, on utilise la glycine.

3. Procédé selon la revendication 1 ou 2, caractérisé en ce que, pour un autre enrichissement en CIG, on reprend le précipité par une solution tampon et on le précipite de nouveau dans les conditions de la revendication 1.